# EUROPEAN PATENT APPLICATION

(11) **EP 0 785 211 A1**
(43) Date of publication of application: **23.07.1997**
(21) Application number: 96400145.7
(22) Date of filing: 22.01.1996
(51) Int. Cl.: C07J 7/00, A61K 31/57, C07J 53/00, C07J 41/00, C07J 13/00, C07J 17/00

(54) **New substituted 19-nor-pregnane derivatives**

(71) Applicant: LABORATOIRE THERAMEX, 98000 Monaco (MC)
(72) Inventor: Maillos, Philippe, de St-Augustin, F-06200 Nice (FR); Verdan, Christina, F-06000 Nice (FR); Delansorne, Rémi, F-06000 Nice (FR); Paris, Jacques, F-06100 Nice (FR); Pascal, Jean-Claude, F-06200 Villefranche sur Mer (FR)
(74) Representative: Gillard, Marie-Louise

(57) **Abstract**

The invention relates to compounds of the formula : 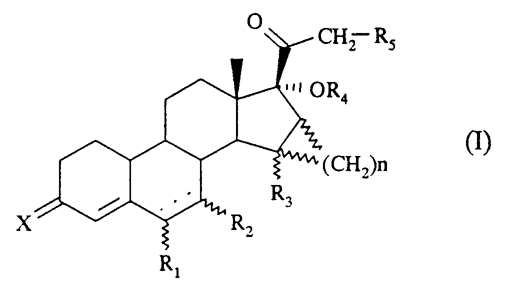 wherein :
each of R₁ and R₂ is hydrogen or a (C₁-C₆)alkyl, R₁ and R₂ being not simultaneously hydrogen ;
R₃ is hydrogen, a (C₁-C₆)alkyl or a (C₁-C₆)alkoxy ;
R₄ is hydrogen, a (C₁-C₆)alkyl or a group -COR₆ where R₆ is a (C₁-C₆)alkyl ;
R₅ is hydrogen or a (C₁-C₆)alkyl ;
n is zero or one ;
X is oxygen or an hydroxyimino group ; and
the dotted line may represent a double bond ; provided that when n = 0, R₃ is hydrogen only if both R₁ and R₂ are a (C₁-C₆)alkyl,
and to pharmaceutical compositions containing them.

These compounds are potent progestogens which are devoid of residual androgenic activity.

## Description

The invention relates to substituted 19-nor-pregnane derivatives, methods of making these compounds and pharmaceutical compositions containing these compounds.

The compounds of this invention have excellent progestative properties while being devoid of residual androgenic activity.

Various substituted 19-nor pregnane derivatives having progestative properties have been described in the literature. For example, GB-A-1 104 968 describes 3-oximino steroids having the formula : 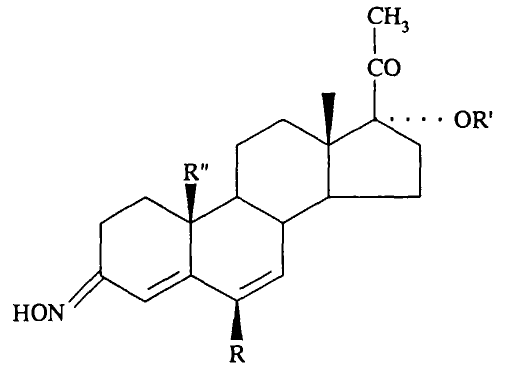 in which R is hydrogen, methyl, ethyl, chlorine, bromine or fluorine ; R' is hydrogen or (C₂-C₁₂)alkanoyl and R'' is hydrogen or methyl ; said compounds being prepared from the 3-oxo derivatives.

DE-A-2 148 261 describes a method of preparing 6α-methyl-19-nor-pregnenes of the formula : 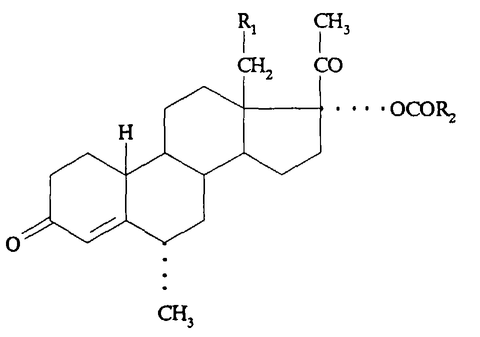 in which R₁ is hydrogen or methyl and R₂ is (C₁-C₉)alkyl.

BE 757 285 relates to pharmaceuticals containing 3,20-dioxo-6α-methyl-17α-acetoxy-19-nor-Δ⁴-pregnene.

In addition, 15,16-methylenesteroids, among which 3-O-Δ⁴-19-nor-17β-Ac-17α-OH-15α,16α-methyleneandrostane and 3-O-Δ⁴-19-nor-17β-Ac-17α-OAc-15α,16α-methyleneandrostane, are described in Chem. Ber. 107, 128-134 (1974).

19-nor progesterone derivatives such as those described above usually exhibit however androgenic side effects.

The Applicant has now found that 19-nor pregnane derivatives which possess at least two substituents in position 6-, 7-, 15- and/or 15,16- display excellent progestative activity and are devoid of residual androgenic activity.

A first aspect of this invention thus encompasses compounds having the structure represented by the following general formula (I): 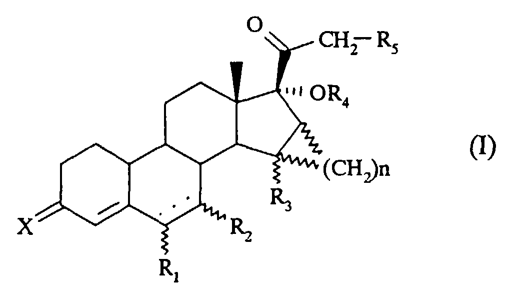 wherein :
each of R₁ and R₂ is hydrogen or a (C₁-C₆)alkyl, R₁ and R₂ being not simultaneously hydrogen ;
R₃ is hydrogen, a (C₁-C₆)alkyl or a (C₁-C₆)alkoxy ;
R₄ is hydrogen, a (C₁-C₆)alkyl or a group -COR₆ where R₆ is a (C₁-C₆)alkyl ;
R₅ is hydrogen or a (C₁-C₆)alkyl ;
n is zero or one ;
X is oxygen or an hydroxyimino group ; and
the dotted line may represent a double bond ; provided that when n = 0, R₃ is hydrogen only if both R₁ and R₂ are a (C₁-C₆)alkyl.

As used herein, the term "alkyl" means a branched of unbranched saturated hydrocarbon radical, such as methyl, ethyl, propyl , isopropyl, butyl, isobutyl, t-butyl, pentyl and hexyl.

As used herein the term "alkoxy" means the group -OR wherein R is alkyl as defined above. Examples include methoxy, ethoxy, propoxy, isopropoxy, butoxy, isobutoxy, t-butoxy, pentoxy and hexyloxy.

As used herein the group -COR₆ wherein R₆ is alkyl as defined above includes, for example, acetyl, propionyl, butyryl, isobutyryl, t-butyryl, valeryl and hexanoyl.

Among the compounds of formula (I), those wherein R₁ and R₃ are a (C₁-C₆)alkyl, R₂ and R₅ are hydrogen, R₄ is a group -COR₆, particularly an acetyl group, and n is zero are preferred. Also preferred are the compounds of formula (I) wherein R₁ is a (C₁-C₆)alkyl, R₃ and R₅ are hydrogen, R₄ is a group -COR₆, particularly an acetyl group and n is one, those where X is oxygen being especially preferred. Further preferred are the compounds of formula (I) wherein R₁ and R₄ are a (C₁-C₆)alkyl, R₂, R₃ and R₅ are hydrogen and n is one, those where X is oxygen being especially preferred. Other preferred compounds of formula (I) include those wherein R₁ and R₂ are a (C₁-C₆)alkyl, R₃ and R₅ are hydrogen, R₄ is a group -COR₆, particularly an acetyl group, and n is zero.

Another aspect of this invention relates to a method of preparing the compounds of formula (I) : these compounds can be made following the Reaction Scheme below wherein R₁, R₂, R₃, R₄, R₅, R₆, n and X have the same meaning as set forth above.

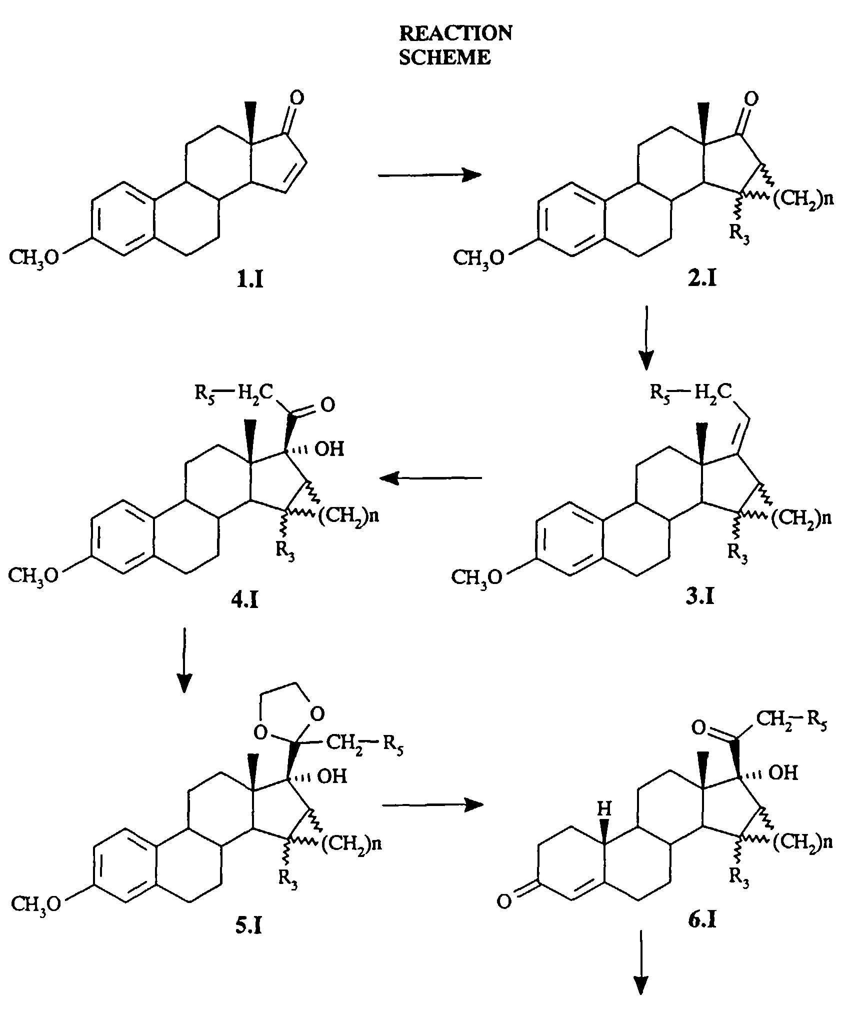

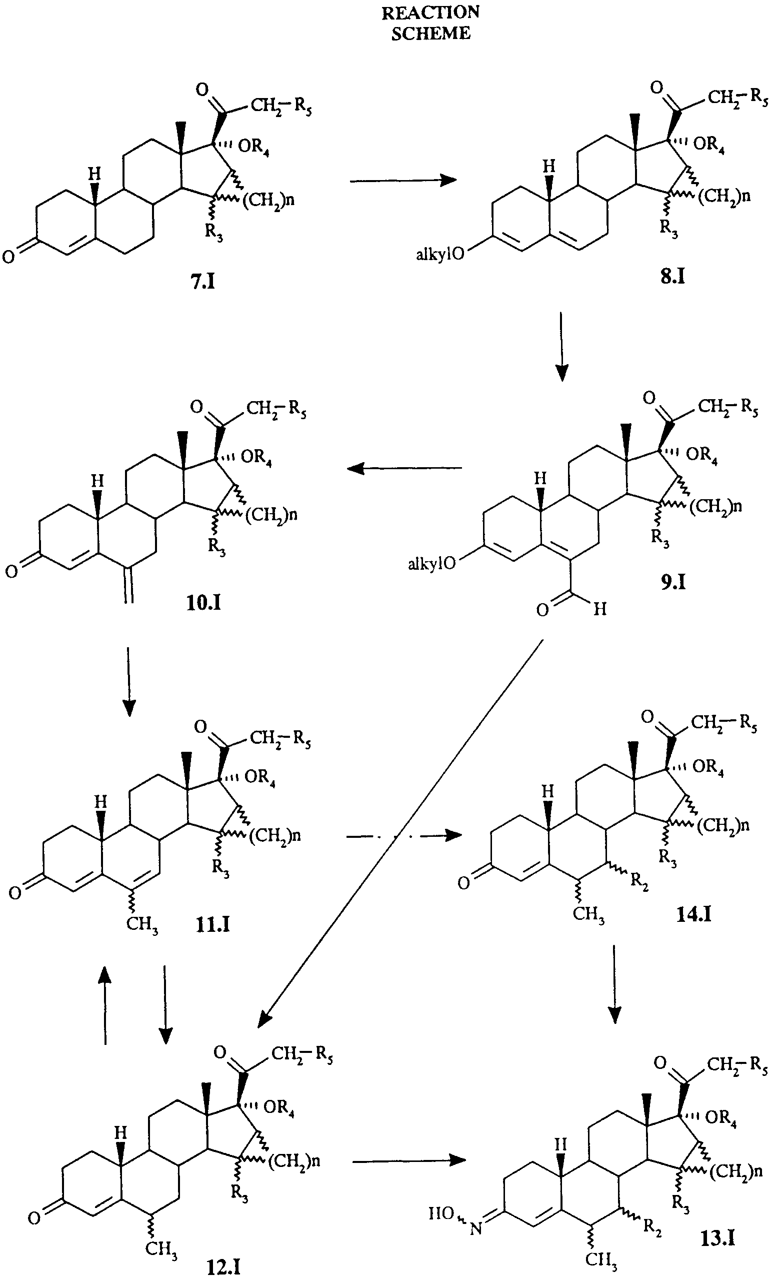

Compounds of formula 1.I are prepared according to procedures described by B.M. Trost et al., J. Am. Chem. Soc. 1976, 98(16), 4887-902, or by J.R. Bull et al., J. Chem. Res. Synop. 1979, (7), 224-5.

Referring to reaction scheme in step 1, compounds of formula 2.I (n = 0, R₃ = alkyl) are prepared using the procedures of M.B. Groën et al., Rec. Trav. Chim. Pays-Bas 1979, 98 (4), 239-49, or R.V. Coombs (US patent 3,766,224).

Compounds of formula 2.I (n = 1, R₃ = H) are prepared by a Corey reaction of a compound of formula 1.I with a dimethylsulfoxonium methylide produced by the reaction of trimethylsulfoxonium iodide with sodium hydride in tetrahydrofurane or dimethylsulfoxide. They can also be prepared by the reaction of diazomethane catalyzed by palladium or copper derivatives.

Compounds of formula 2.I (n = O, R₃ = alkoxy) can be prepared using the procedure described by E.N. Cantrall et al., J. Org. Chem. (1964), 29, 64.

Compounds of formula 2.I are condensed with an alkyl-triphenyl-phosphonium bromide in a dimethylsulfoxide/sodium hydride mixture at a temperature from about 10°C to 40°C, preferably at 20°C.

After about 1 to 24 hours, preferably 8 to 12 hours, products of formula 3.I are separated from the reaction mixture by precipitation on water followed by crystallization in an alcohol, preferably methanol or ethanol.

Compounds of formula 3.I are oxidized by a complex of N-methyl-morpholine oxide-hydrogen peroxide catalysed by osmium tetraoxide, or N-triethylamine oxide-hydrogen peroxide catalysed by osmium tetraoxide in tert-butanol at a temperature from about 0°C to 40°C, preferably at 20°C for about 2 to 12 hours, preferably 6 hours. The reaction mixture is treated with a solution of sodium sulfite (Na₂SO₃) and compounds of formula 4.I are obtained by precipitation on water or flash-chromatography. Other oxidizing agents such as KMNO₄ in an acetone/water mixture can be used in this step following a process described by A.J. Fatiadi (Synthesis 1987, 85).

Compounds of formula 5.I are then dissolved in toluene to which is added 1 to 10 molar equivalents of ethylene glycol, preferably 5 molar equivalents, triethylorthoformate and a catalytic amount of p-toluenesulfonic acid. The reaction mixture is stirred at a temperature of about 20°C to 80°C, preferably 40°C, for about 2 to 8 hours, preferably 6 hours. Then, the reaction mixture is cooled and poured into iced water and extracted with a suitable organic solvent, such as toluene, diethyl ether and the like. When the solvent is removed under reduced pressure, a residue is formed which can be purified by crystallization in an alcohol by flash-chromatography to yield compounds of formula 5.I.

Compounds of formula 5.I are submitted to Birch reduction following the procedure of A.J. Birch et al., J. Chem. Soc. (1949), 2531, yielding compounds of formula 6.I.

The tertiary hydroxy group can be optionally esterified by known processes used for esterification in steroid chemistry or etherified by an alkyl halide according to conventional methods of Williamson ether synthesis such as described by B. G. Zupancic et al., Synthesis (1979), 123 or by D.R. Benedict et al., Synthesis (1979), 428-9 to give compounds of formula 7.I (R₄ = H, -COR₆ or alkyl).

The corresponding alkyl enol ethers of formula 8.I are obtained by reaction with a trialkylorthoformate, preferably triethylorthoformate in an alcohol such as methanol, ethanol, propanol or n-butanol, preferably ethanol with a catalytic amount of toluene sulfonic acid. The reaction temperature can be, for example, room temperature to the boiling temperature of the reaction mixture.

The alkyl enol ethers are reacted with a Vilsmeier-Hack formylating agent in DMF at a temperature below 0°C, preferably -10°C, giving compounds of general formula 9.I.

Sodium borohydride reduction of compounds 9.I in a mixture of DMF and CH₃OH following by dehydration with a strong mineral acid such as H₂SO₄, HCl or HBr, preferably sulfuric acid, yield compounds of formula 10.I. Alternatively, compounds of formula 9.I can be directly converted to 12.I by a process similar to that described by D. Burn et al., Tetrahedron (1965), 21, 1619-24.

Treatment of 10.I with palladium-on-charcoal in refluxing alcohol such as methanol, ethanol or isopropanol gives, after filtration of the catalyst and crystallization in an appropriate solvent compounds of formula 11.I. Alternatively, compounds of formula 10.I can be directly converted to compounds of formula 12.I by hydrogenation. The pure isomers are obtained by crystallization or by preparative HPLC.

Reaction of compounds of formula 12.I with an excess of hydroxylamine hydrochloride in a mixture of an alcohol, preferably ethyl alcohol, or dioxane, and pyridine at a temperature between 20°C to reflux of the solvent, preferably 60°C, leads to compounds of formula 13.I. The E and Z hydroxyimino derivatives are obtained by crystallization in a suitable solvent or by flash-chromatography.

Addition of a lithium dialkyl cuprate LiCu(R₂)₂, or of the corresponding alkylmagnesium halide under copper catalysis (for example CuI or CuCN) to compounds of formula 11.I following by isomerisation of the double bond in acidic condition yield compounds of formula 14.I. These can be converted to 13.I by addition of hydroxylamine.

The compounds of this invention are potent progestogens which are devoid of androgenic side effects ; they are therefore useful for treating a variety of gynaecological disorders related to an oestrogen/progesterone imbalance in premenopausal women, for example : abnormal uterine bleeding (oligomenorrhea, amenorrhea, functionnal uterine hemorrhage...), premenstrual tension, dysmenorrhea, benign breast disease, breast tumors, premenstrual complaints such as headache, depression, water retention, mastodynia or mood alteration.

Due to their pharmacological properties, in particular their potent antigonadotropic activity, they can also be used in several other indications, such as for example the inhibition of oestrogen transformation of normal endometrium to hyperplasic or malignant states, or for the inhibition of gonadotropic/gonadal secretions (for example in the treatment of endometriosis and polycystic ovary syndrome in women and of prostate diseases in men).

The invention compounds can also be used alone as contraceptive agents in both sexes, or in combination with an oestrogen, such as for example oestradiol or ethinyl oestradiol, in contraceptive formulations for premenopausal women ; they can also be used alone or in combination with an oestrogen in hormonal replacement therapy for postmenopausal women.

The progestative activity of the compounds of formula (I) can be evaluated in two specific experimental models : the affinity for the progesterone receptor (PR) *in vitro*, and the endometrial tranformation of the rabbit uterus *in vivo*.

Human PR are readily available in high amounts from the T47-D cell line in culture (M.B. Mockus et al., *Endocrinology*, 1982, 110 : 1564-1571). Relative binding affinities (RBA) for the human T47-D cell PR are determined as previously described (J. Botella, et al., *J. Steroid Biochem. Molec. Biol*., 1994, 50 : 41-47) using [³H]-ORG 2058 as the labelled specific ligand (G. Fleischmann et al., Biochim. Biophys. Acta, 1978, 540 : 500-517) and nomegestrol acetate as the non-radioactive reference progestin. Competitive incubations were performed against 2 nM of [³H]-ORG 2058 for 3 hours at 4°C with six different concentrations of non-labelled steroid, chosen between 4 and 256 nM following a 1/2ⁿ dilution scheme. Displacement curves were fitted for each experiment, and the concentration inhibiting 50% of the specific binding of [³H]-ORG 2058 was calculated for each curve (IC₅₀).

The affinity of test compounds of the invention for human PR is given in Table 1 below :

**Table 1**

| Relative binding affinity to human T47-D cell progesterone receptor | | | |
|---|---|---|---|
| Progestin | IC₅₀^{(a)} in nM | (n) | RBA |
| Nomegestrol acetate | 16.4 ± 1.3 | (13) | 100% |
| Example 1 | 38.0 ± 10.3 | (4) | 43% |
| Example 2 | 28.8 ± 5.6 | (3) | 57% |
| Example 6 | 21.1 ± 6.0 | (4) | 78% |
| Example 7 | 19.7 ± 3.0 | (4) | 83% |
| Example 8 | 21.1 ± 6.0 | (4) | 78% |
| Example 9 | 32.3 ± 5.3 | (4) | 51% |
| Example 11 | 25.5 ± 5.3 | (3) | 64% |

| | | | |
|---|---|---|---|
| (a) mean ± s.e.m. ; | | | |
| (n) number of experiments | | | |

One specific pharmacological test has been standardized *in vivo* for the detection and quantitation of pseudogestagenic activity since the mid-30's : it is based on the property of the uterus of estrogen-primed immature female rabbits to respond to very slight amounts of progestin by a typical endometrial transformation into a densely packed and interlaced epithelial network called "dentelle". The original test schedule, which includes 6 days of estrogen priming (total subcutaneous dose of 30 µg/rabbit of estradiol benzoate) followed by 5 days of progestative treatment, was designed as early as 1930 by C. Clauberg, *Zentr. Gynäkol*. 1930, 54 : 2757-2770. The semi-quantitative scale used to grade the intensity of the microscopical appearance of the "dentelle" was set up by M.K. McPhail, *J. Physiol (London)*, 1934, 83, 145-156. This overall Clauberg-McPhail procedure has been extensively used to screen steroids for putative progestative activity *in vivo* and is still part of the basic hormonal profile of any new progestin such as norgestimate (A. Phillips et al., *Contraception*, 1987, 36, 181-192) or desogestrel (J. Van der Vies et al.,*Arzneim. Forsch./Drug Res*. 1983, 33, 231-236).

The progestative potency is inversely related to the dose needed to induce a half-maximal stimulation of the "dentelle", i.e. to record a mean McPhail grade equal to 2. This ED₅₀ is deduced for the dose-response curve and expressed in total dose/rabbit/5 days. All compounds were tested for activity only following oral administration. The maximal administred dose was 0.75 mg, roughly corresponding to 5 times the ED₅₀ of nomegestrol acetate, a potent orally active 19-norprogesterone-derived progestin (J. Paris et al., *Arzneim. Forsch./Drug Res*., 1983, 33, 710-715).

The oral activity of test compounds of the invention is given in Table 2 below :

**Table 2**

| Clauberg-McPhail test by oral administration | | |
|---|---|---|
| Progestin | ED₅₀ (mg/rabbit/5 days) | Relative activity |
| Example 1 | > 0.750 | < 18% |
| Example 2 | 0.080 | 165% |
| Example 6 | > 0.750 | < 18% |
| Example 7 | 0.245 | 54% |
| Example 8 | 0.133 | 99% |
| Example 10 | 0.600 | 22% |
| Example 11 | 0.100 | 132% |
| Nomegestrol acetate | 0.132 ± 0.013^{(a)} (0.108/0.150/0.138) | 100% |

| | | |
|---|---|---|
| (a) mean ± s.e.m. of 3 different experiments in which the cited examples were tested in parallel | | |

The residual androgenic potential is an important feature to evaluate for any new progestin, because it is highly predictive of androgenic side-effects in women. One pharmacological model of androgenic activity has been standardized to screen steroids or related compounds in immature castrated male rats, using the hypertrophy of the ventral prostate and seminal vesicles as the endpoint, following 10 daily administrations (R.I. Dorfman, in *Methods in Hormone Research*, volume 2, LONDON, Academic Press, 1962 : 275-313 ; A.G. Hilgar et D.J. Hummel, *Androgenic and Myogenic Endocrine Bioassay Data*, edited by the U.S. Department of Health, Education and Welfare, WASHINGTON D.C., 1964).

Medroxyprogesterone acetate is a 6α-methylpregnene derivative which, besides its main progestative activity, is well-known for its weak androgenic properties (M. Tausk et J. de Visser, In *International Encyclopedia of Pharmacology and Therapeutics, Section 48* : Progesterone, Progestational Drugs and Antifertility Agents, volume II, OXFORD, Pergamon Press, 1972 : 35-216) ; it was therefore chosen as a reference compound in the testing for residual androgenic activity of test examples according to the invention.

The compounds of examples 2 and 7 were tested for residual androgenic properties in the immature castrated male rat model by oral administration (PO), in comparison with medroxyprogesterone acetate, in two different experiments (ventral prostate and seminal vesical hypertrophy), as described above ; testosterone was used as a standard androgenic agent by subcutaneous injection (SC).

**Table 3**

| Residual androgenic activity of the compound of example 2 | | | |
|---|---|---|---|
| Steroid | Dose (mg/animal/day) | Ventral Prostate (mg) | Seminal Vesicle (mg) |
| Castrated controls | - | 14.6 ± 1.4 | 10.4 ± 0.4 |
| Testosterone, SC | 0,05 | 85.1 ± 5.9** | 85.9 ± 6.8** |
| Medroxyprogesterone acetate, PO | 20 | 23.1 ± 1.1* | 22.1 ± 1.1** |
| Example 2, PO | 20 | 13.0 ± 1.1 ns | 12.1 ± 0.7 ns |
| mean ± s.e.m. of 8 animals per group ; | | | |

| | | | |
|---|---|---|---|
| * p < 0.05 and | | | |
| ** p < 0.01 | | | |
| ns : not statistically different from controls. | | | |

**Table 4**

| Residual androgenic activity of the compound of example 7 | | | |
|---|---|---|---|
| Steroid | Dose (mg/animal/day) | Ventral Prostate (mg) | Seminal Vesicle (mg) |
| Castrated controls | - | 12.0 ± 0.9 | 12.3 ± 0.7 |
| Testosterone, SC | 0,05 | 90.4 ± 4.4*** | 90.3 ± 6.3** |
| Medroxyprogesterone acetate, PO | 20 | 29.1 ± 1.4*** | 19.9 ± 1.8** |
| Example 7, PO | 20 | 16.5 ± 0.9 ** | 13.9 ± 0.8 ns |
| mean ± s.e.m. of 8 animals per group ; | | | |

| | | | |
|---|---|---|---|
| * p < 0.05 and | | | |
| ** p < 0.01 | | | |
| ns : not statistically different from controls. | | | |

The compound of example 7 exhibits a very limited androgenic potential, which reachs statistical significance on ventral prostate weights but not on seminal vesicle weights (Table 3), and the compound of example 2 is totally devoid of any activity on the growth of male accessory sex organs, up to at least 20 mg/animal/day (Table 4). By comparison, medroxyprogesterone acetate reproducibly induces approximately twice the values obtained for the above compounds at the same dose (Tables 3 and 4).

Thus, the compounds of the present invention are excellent progestogens with no residual androgenic activity.

Thus according to another aspect, the invention relates to pharmaceutical compositions containing an effective amount of a compound of formula (I), mixed with suitable pharmaceutical excipients. Said compositions may further comprise an effective amount of an oestrogen.

Another aspect of the invention comprises a method of treating or preventing gynaecological disorders ; a method of inhibiting oestrogen transformation of endometrium ; and a method of inhibiting gonadotropic/gonadal secretions.

The compounds of this invention are administered at a therapeutically effective dosage, i.e., a dosage sufficient to provide treatment for the disease states previously described. Administration of the active compounds described herein can be via any of the accepted modes of administration for agents that serve similar utilities.

Generally, a daily dose is from 0.001 to 1 mg/kg of body weight per day of the compound of formula (I). Most conditions respond to a treatment comprising a dosage level on the order of 0.002 to 0.2 mg/kg of body weight per day. Thus, for administration to a 50 kg person, the dosage range would be about 2 mg per day, preferably between about 0.5 to 5 mg per day.

Depending on the specific disease state, administration can be via any accepted systemic route, for example, via oral route, parenteral route such as intravenous, intramuscular, subcutaneous or percutaneous route, or vaginal, ocular or nasal route, in the form of solid, semi-solid or liquid dosage forms, such as for example, tablets, suppositories, pills, capsules, powders, solutions, suspensions, cream, gel, implant, patch, pessary, aerosols, emulsions or the like, preferably in unit dosage forms suitable for simple administration of precise dosages. The compositions will include a conventional pharmaceutical carrier or vehicle and an active compound of formula (I) and, in addition, may include other medicinal agents, pharmaceutical agents, carriers, adjuvants, etc.

If desired, the pharmaceutical composition to be administered may also contain minor amounts of non-toxic auxiliary substances such as wetting or emulsifying agents, pH buffering agents and the like, such as for example sodium acetate, sorbitan monolaurate, triethanolamine oleate, etc.

The compouds of this invention are generally administered as a pharmaceutical composition which comprises a pharmaceutical vehicle in combination with a compound of formula (I). The level of the drug in a formulation can vary within the full range employed by those skilled in the art, e.g., from about 0.01 weight percent (wt%) to about 99.99 wt% of the drug based on the total formulation and about 0.01 wt% to 99.99 wt% excipient.

The preferred manner of administration, for the conditions detailed above, is oral administration using a convenient daily dosage regimen which can be adjusted according to the degree of affliction. For such oral administration, a pharmaceutically acceptable, non-toxic composition is formed by the incorporation of the compound of formula (I) in any of the normally employed excipients, such as, for example, pharmaceutical grades of mannitol, lactose, starch, magnesium stearate, sodium saccarine, talcum, cellulose, glucose, gelatin, sucrose, magnesium carbonate, and the like. Such compositions take the form of solutions, suspensions, tablets, pills, capsules, powders, sustained release formulations and the like. Such compositions may contain between 0.01 wt% and 99.99 wt% of the compound of formula (I).

Preferably the compositions will take the form of a sugar coated pill or tablet and thus the composition will contain, along with the active ingredient, a diluent such as lactose, sucrose, dicalcium phosphate, and the like ; a disintegrant such as starch or derivatives thereof ; a lubricant such as a magnesium stearate and the like ; and a binder such as a starch, polyvinylpyrrolidone, gum acacia, gelatin, cellulose and derivatives thereof, and the like.

### EXAMPLES

The following examples are given to enable those skilled in the art to more clearly understand and to practice the present invention. They should not be considered as a limitation of the scope of the invention but merely as being illustrative and representative thereof. In these examples, the following abbreviations are used :
- MeOH :: methanol
- EtOH :: ethanol
- Ac :: acetyl
- OAc :: acetoxy
- AcOEt :: ethyl acetate
- DMF :: dimethylformamide
- THF :: tetrahydrofuran
- Pd/C :: palladium -on- charcoal
- Pd/Al₂O₃ :: palladium -on- alumina
- DMSO :: dimethylsulfoxide
- DDQ :: 2,3-dichloro-5,6-dicyanobenzoquinone
- s :: singlet
- d :: doublet
- t :: triplet
- q :: quadruplet
- bs :: broad singlet
- dd :: doubled doublet
- m :: multiplet

### EXAMPLE 1

### 17α-acetoxy-6,15β-dimethyl-3,20-dioxo-19-nor-pregna-4,6-diene (11.I.A : R₁ = CH₃, R₃ = CH₃, R₄ = Ac, R₅ = H, n = 0)

A/ 3-methoxy-15β-methyl-19-nor-pregna-1,3,5(10)-17(20)-tetraene (Z or E) (3.I.a : R₃ = CH₃, R₅ =H, n = 0)
   Sodium hydride (60 % in mineral oil, 4.88 g, 122 mmol.) was added to a round-bottomed flask fitted with a magnetic stirrer bar. The sodium hydride dispersion was washed with dry hexane to remove the mineral oil and 200 mL of dry dimethylsulfoxide was added to the flask. Ethyl-triphenyl-phosphonium bromide (37.77 g, 101 mmol.) was added to the dispersion and the solution was stirred at 20°C under nitrogen for 2 hours. Then 3-methoxy-15β-methyl-estra-1,3,5(10)-triene-17-one (2.I.a) obtained as described in "Recueil des travaux chimiques des Pays Bas" (vol 98, n°4, 1979) was added to the solution and the mixture was stirred for 22 hours at room temperature. The mixture was poured into 2 L of iced water and stirred for 1 hour, then filtered and recrystallized from ethanol to give 3.I.a (9.17 g, yield : 73 %), mp : 97°C.
   ¹H-NMR (CDCl₃,δ) : 0.99 (d, 3H) ; 1.02 (s, 3H) ; 1.69 (d, 3H) ; 3.70 (s, 3H) ; 6.65 (d, 1H) ; 6.75 (dd, 1H) ; 7.21 (d, 1H).
B/ 17α-hydroxy-3-methoxy-15β-methyl-20-oxo-19-nor-pregna-1,3,5(10)-triene (4.I.a : R₃ = CH₃, R₅ = H, n = 0)
   A solution of osmium tetraoxide in pyridine (20 g/L ; 11 mL) was added to a suspension of compound 3.I.a (9.17 g ; 29.5 mmol.) in 200 mL of 2-hydroxy-2-methylpropane. Then, a complex of N-methylmorpholine oxide-hydrogen peroxide prepared as described in Reagents for Organic Synthesis, Fieser and Fieser vol. 1, p. 690 (4.9 g, 29 mmol.) was added. The solution was stirred for 6 hours. The reaction mixture was treated with 150 mL of 0.5 % sodium sulfite, stirred for 1 hour and filtered. The filtrate was evaporated to dryness and the crude product was flash-chromatrographed using toluene/ethyl acetate (95/5) as eluting solvent to give 5.7 g of 4.I.a (yield : 56 %), mp : 61°C.
   IR (KBr, cm⁻¹) : 3335 νOH ; 1687 νC = O
   ¹H-NMR (CDCl3,δ) : 0.91 (s, 3H) ; 1.08 (d, 3H) ; 2.31 (s, 3H) ; 2.94 (s, 1H) ; 3.48 (d, 2H) ; 3.78 (s, 3H) ; 6.63 (d, 1H) ; 6.71 (dd, 1H) ; 7.20 (d, 1H).
C/ 20-(ethylenedioxy)-17α-hydroxy-3-methoxy-15β-methyl-19-nor-pregna-1,3,5 (10) triene (5.I.a : R₃ = CH₃, R₅ = H, n = 0)
   p-toluenesulfonic acid (600 mg, 3.12 mmol.), compound 4.I.a (12.06 g, 35.2 mmol.), ethylene glycol (250 mL, 6.98 mol.), triethylorthoformate (50 mL, 297 mmol.) in toluene (120 mL) were stirred together for 5 hours. Precipitation on water and extraction with toluene gave, after evaporation, a crude product which was chromatographed using a mixture of toluene/ethyl acetate (95/5) as eluting solvent to give 12 g of 5.I.a (yield: 88 %), mp : 113°C.
   IR (KBr, cm⁻¹) : 3574 νOH
   ¹H-NMR (CDCl₃,δ) : 0.99 (s, 3H) ; 0.99 (d, 3H) ; 1.40 (s, 3H) ; 3.73 (s, 3H) ; 6.19 (dd, 1H) ; 6.51 (d, 1H) ; 7.19 (d, 1H).
D/ 17α-hydroxy-15β-methyl-3,20-dioxo-19-nor-pregna-4-ene (6.I.a : R₃ = CH₃, R₅ = H, n = 0)
   Compound 5.I.a (12.36 g, 32 mmol.) was dissolved in 160 mL of dry THF and 12 mL of ethanol was added. The solution was then poured into ammoniac (250 mL) at -30°C. Lithium (2.4 g, 345 mmol.) was added portionwise at -30°C, and the dark solution was stirred at -30°C for 1 hour. Ethanol (12 mL) was added and the solution was stirred for 15 hours at room temperature. Then ammoniac was removed, methanol (250 mL) and a solution of 50 % hydrogen chloride (HCl, 100 mL) were added, followed by an extraction with dichloromethane. The organic layer was dried (Na₂SO₄) and evaporated under reduced pressure giving 10.17 g of crude 6.I.a (yield : 96 %), mp : 218°C.
   UV_{λmax} : 239 nm
   IR (KBr, cm⁻¹): 3439 νOH ; 1701 νC = O ; 1651 νC = O conjugated
   ¹H-NMR (CDCl₃,δ) : 1.88 (s, 3H) ; 2.01 (d, 3H) ; 3.21 (s, 3H) ; 4.00 (s, 1H) ; 6.76 (s, 1H).
E/ 17α-acetoxy-15β-methyl-3,20-dioxo-19-nor-pregna-4-ene (7.I.a : R₃ = CH₃, R₄ = Ac, R₅ = H, n = 0)
   p-toluenesulfonic acid (1.2 g, 6.24 mmol.) was added to a suspension of 6.I.a (10.7 g, 30 mmol.) in a mixture of acetic acid (80 ml, 1.38 mmol.) and acetic anhydride (40 mL, 42 mmol.). The solution was stirred for 18 hours. The solution was poured into water. Extraction with CH₂Cl₂ gave an oily product which was treated in a mixture of CH₂Cl₂ (100 mL) and methanol (100 mL) with concentrated HCl (8 mL), and stirred for 18 hours. Water was added to the mixture and the product was extracted with CH₂Cl₂ and chromatographed, using toluene/ethyl acetate (6/4) as eluting solvent to give 7.4 g of 7.I.a (yield : 67 %), mp : 243°C.
   UV_{λmax} : 238 nm
   IR (KBr, cm⁻¹) : 1733 νC = O ; 1713 νC = O ; 1656 νC = O conjugated
   ¹H-NMR (CDCl₃,δ) : 0.84 (s, 3H) ; 1.03 (d, 3H) ; 2.06 (s, 3H) ; 2.12 (s, 3H) ; 5.86 (s, 1H).
F/ 17α-acetoxy-3-ethoxy-15β-methyl-20-oxo-19-nor-pregna-3,5(6)-diene (8.I.a: R₃ = CH₃, R₄ = Ac, R₅ = H, n = 0, alkyl = ethyl)
   p-toluenesulfonic acid (40 mg, 0.21 mmol.), 7.I.a (7.34 g, 19.7 mmol.), triethyl orthoformate (7 mL, 42 mmol.) and ethanol (60 mL) were stirred together for 4 hours. Then triethylamine (TEA) (4 mL) was added and the entire solution was poured into 500 mL of an ice/water mixture, stirred for 1 hour, filtered and recrystallized from ethanol containing a small amount of TEA to give 1.87 g of 8.I.a (yield : 23 %), mp : 232°C.
   IR (KBr, cm⁻¹) : 1732 νC = O ; 1708 νC = O ;
   UV_{λmax} : 241 nm
G/ 17α-acetoxy-3-ethoxy-6-formyl-15β-methyl-20-oxo-19-nor-pregna-3,5 (6)-diene (9.I.a : R₃ = CH₃, R₄ = Ac, R₅ = H, n = 0, alkyl = ethyl)
   In a round-bottomed flask POCl₃ (0.6 mL, 6.3 mmol.) was added to DMF (2.88 mL, 37 mmol.) at -10°C. This solution was added to a suspension of compound 8.I.a (1.8 g, 4.4 mmol.) in DMF (10 mL) at -12°C. The red mixture was stirred for 2 hours at -12°C, and treated with 175 mg of NaHCO₃ followed by a solution of CH₃COOK (12 mL) and water (30 mL). The product was extracted with CH₂Cl₂, evaporated in vacuo to give 1.82 g of 9.I.a (yield : 94 %), mp : 171°C.
   UV λₘₐₓ : 324 and 221 nm
   IR (KBr, cm⁻¹) : 1734 νC = O ; 1652 νC = O ; 1608 νC = O
   ¹H-NMR (CDCl₃,δ) : 0.81 (s, 3H) ; 1.01 (d, 3H) ; 1.48 (t, 3H) ; 2.08 (s, 3H) ; 2.11 (s, 3H) ; 6.37 (s, 1H) ; 10.23 (s, 1H).
H/ 17α-acetoxy-15β-methyl-6-methyliden-3,20-dioxo-19-nor-pregna-4-ene (10.I.a: R₃ = CH₃, R₄ = Ac, R₅ = H, n = 0)
   A round-bottomed flask was fitted with a magnetic stirred bar, then compound 9.I.a (1.82 g, 4.2 mmol.), DMF (6 mL) and EtOH (6 mL) were added. NaBH₄ (170 mg, 4.5 mmol.) was added portionwise. Then, a solution of 50 % sulfuric acid (0.8 mL) was added to the mixture. The solution was poured into iced water (200 mL) and the precipitate was filtered to give 1.52 g of crude 10.I.a (yield : 93 %), mp : 200°C.
   UV_{λmax} : 263 nm
   IR (KBr, cm⁻¹) : 1732 νC = O ; 1714 νC = O ; 1659 νC = O
   ¹H-NMR (CDCl₃,δ) : 0.83 (s, 3H) ; 1.05 (d, 3H) ; 2.04 (s, 3H) ; 2.10 (s, 3H) ; 4.97 (s, 1H); 5.19 (s, 1H) ; 6.10 (s, 1H).
I/ 17α-acetoxy-6,15β-dimethyl-3,20-dioxo-19-nor-pregna-4,6-diene (11.I.a : R₁ = CH₃, R₃ = CH₃, R₄ = Ac, R₅ = H, n = 0)
   A mixture of 10.I.a (980 mg, 2.54 mmol.), Pd/C (5 % with 50 % H₂O) (1.6 g) and MeOH (180 mL) was refluxed during 30 min. The solution was filtered to remove the catalyst. Then, the crude product obtained by evaporating the solvent was flash-chromatographed using toluene/ethyl acetate as eluting solvent (7/3) to give 480 mg of 11.I.a (yield : 49 %), mp : 244°C.
   UV λₘₐₓ : 292 nm
   IR (KBr, cm⁻¹) : 1736 νC = O ; 1710 νC = O ; 1654 νC = O
   ¹H-NMR (CDCl₃,δ) : 0.88 (s, 3H) ; 1.08 (d, 3H) ; 1.88 (s, 3H) ; 2.08 (s, 3H) ; 2.1 (s, 3H) ; 5.95 (s. 1H) ; 6.15 (s, 1H).

### EXAMPLE 2

### 17α-acetoxy-6α,15β-dimethyl-3,20-dioxo-19-nor-pregna-4-ene (12.I.a : R₃ = CH₃, R₄ = Ac, R₅ = H, n = 0)

Compound 11.I.a (360 mg, 0.93 mmol.) was hydrogenated in 20 mL of EtOH with 0.3 mL of cyclohexene under stirring over 100 mg of Pd/Al₂O₃. The reaction was complete after 1 hour at reflux. The palladium catalyst was filtered. The crude product was obtained by removing the solvent. Crystallization of the crude product in isopropylether gave 100 mg of the title compound (yield : 28 %), mp : 203°C.
IR (KBr, cm⁻¹) : 1734 νC = O ; 1714 νC = O ; 1661 νC = O
δH (CDCl₃) : 0.85 (s, 3H) ; 1.08 (d, 3H) ; 1.15 (d, 3H) ; 2.05 (s, 3H) ; 2.1 (s, 3H) ; 5.9 (s, 1H)

### EXAMPLE 3

### 17α-acetoxy-6β,15β-dimethyl-3,20-dioxo-19-nor-pregna-4-ene (12.I.b : R₃ = CH₃, R₄ = Ac, R₅ = H, n = 0)

Preparative HPLC of the mother liquid of 12.I.a yielded the 6β-methyl isomer.

### EXAMPLES 4-5

### 17α-acetoxy-6α,15β-dimethyl-3-(E and Z)-hydroxyimino-20-oxo-pregna-4-ene (13.I.a : R₂ = H, R₃ = CH₃, R₄ = Ac, R₅ = H, n = 0)

A mixture of 12.I.a (1 g, 2.58 mmol.), hydroxylamine hydrochloride (250 mg, 3.1 mmol.), pyridine (0.4 mL, 4.9 mmol.) and methanol (10 mL) was refluxed for 2 hours. Evaporation of the solvent gave a crude product which was chromatographed using a mixture of toluene/ethyl acetate (9/1) as eluting solvent to give 560 mg of 17α-acetoxy-6α,15β-dimethyl-3-E- hydroxyimino-20-oxo-pregna-4-ene (yield : 54 %), mp : 235°C (example 4).
IR (KBr, cm⁻¹) : 3331 νOH ; 1741 and 1702 νC = O
¹H-NMR (CDCl₃, δ) : 0.83 (s, 3H) ; 1.04 (d, 3H) ; 1.13 (d, 3H) ; 2.04 (s, 3H) ; 2.09 (s, 3H) ; 5.92 (s, 1H).
and 80 mg of 17α-acetoxy-6α,15β-dimethyl-3-Z-hydroxyimino-20-oxo-pregna-4-ene (yield : 8 %), mp: 122°C (example 5).
IR (KBr, cm⁻¹) : 2923 νOH ; 1736 and 1706 νC = O
¹H-NMR (CDCl₃, δ) : 0.82 (s, 3H) ; 1.03 (d, 3H) ; 1.10 (d, 3H) ; 2.06 (s, 3H) ; 2.11 (s, 3H) ; 6.60 (s, 1H).

### EXAMPLE 6

### 17α-acetoxy-6-methyl-15β,16β-methylene-3,20-dioxo-19-nor-pregna-4,6-diene (11.I.b : R₃ = H, R₄ = Ac, R₅ = H, n = 1)

A/ 3-methoxy-15β,16β-methylene-19-nor-pregna-1,3,5(10),17(20)-tetraene (3.I.b: R₃ = H, R₅ = H, n = 1)
   3.I.b was prepared from 3-methoxy-15β,16β-methylene-19-nor-17-oxo-pregna-1,3,5(10)-triene obtained as described by O. Schmidt et al., Chem. Ber. 101, 939 (1968) following the process described for 3.I.a (yield : 96 %), mp : 76°C.
      IR (KBr, cm⁻¹) : 1605 νC = C
      ¹H-NMR (CDCl₃,δ) : 0.5 (m, 1H) ; 1 (s, 3H) ; 1.7 (d, 3H) ; 3.75 (s, 3H) ; 5.38 (q, 1H) ; 6.75 (m, 2H) ; 7.1 (d, 1H).
B/ 17α-hydroxy-3-methoxy-15β,16β-methylene-20-oxo-19-nor-pregna-1,3,5(10)-triene (4.I.b : R₃ = H, R₅ = H, n = 1)
   4.I.b was prepared from 3.I.b as described for 4.I.a (yield : 47 %), mp : 134°C. IR (KBr, cm⁻¹) : 3508 νO-H ; 1693 νC = O
      ¹H-NMR (CDCl₃,δ) : 0.7 (m, 1H) ; 0.8 (s, 3H) ; 2.4 (s, 3H) ; 3.1 (s, 1H) ; 6.7 (m, 2H) ; 7.1 (d, 1H).
C/ 20-(ethylenedioxy)-17α-hydroxy-3-methoxy-15β,16β-methylene-19-nor-pregna-1,3,5 (10)-triene (5.I.b: R₃ = H, R₅ = H, n = 1)
   5.I.b was obtained as described for 5.I.a from compound 4.I.b (yield : 95 %), mp : 166°C.
      IR (KBr, cm⁻¹) : 3507 νOH ; 1280 νC-O
      ¹H-NMR (CDCl₃,δ) : 0.45 (m, 1H) ; 1.05 (s, 3H) ; 1.5 (s, 3H) ; 3.75 (s, 3H) ; 5 (m, 4H) ; 6.67 (m, 2H) ; 7.1 (d, 1H).
D/ 17α-hydroxy-15β,16β-methylene-19-nor-3,20-dioxo-pregna-4-ene (6.I.b: R₃ = H, R₅ = H, n = 1)
   6.I.b was prepared from 5.I.b using the same process than for 6.I.a (yield : 63 %), mp : 175°C.
      IR (KBr, cm⁻¹) : 3461 νOH ; 1706 νC = O (20) ; 1661 νC = O (3) ; 1609 νC =C
      ¹H-NMR (CDCl₃,δ) : 0.65 (m, 1H) ; 0.85 (s, 3H) ; 2.4 (s, 3H) ; 3.05 (s, 1H) ; 5.85 (s, 1H).
E/ 17α-acetoxy-15β,16β-methylene-3,20-dioxo-19-nor-pregna-4-ene (7.I.b : R₃ = H, R₄ = Ac, R₅ = H, n = 1)
   7.I.b was obtained from 6.I.b as described for compound 7.I.a (yield : 79 %), mp : 207°C.
      IR (KBr, cm⁻¹) : 1732 νC = O ; 1715 νC = O ; 1659 νC = O ; 1615 νC = C
      ¹H-NMR (CDCl₃,δ) : 0.65 (m, 1H) ; 0.8 (s, 3H) ; 2.05 (s, 3H) ; 2.3 (s, 3H) ; 5.89 (s, 1H)
F/ 17α-acetoxy-3-ethoxy-15β,16β-methylene-20-oxo-19-nor-pregna-3,5(6)-diene (8.I.b : R₃ = H, R₄ = Ac, R₅ = H, alkyl = ethyl, n = 1)
   8.I.b was prepared from 7.I.b using the same process than for 8.I.a (yield : 69 %), mp : 164°C.
      IR (KBr, cm⁻¹) : 1735 νC = O ; 1707 νC = O ; 1646-1619 νC
      ¹H-NMR (CDCl₃,δ) : 0.65 (m, 1H) ; 0.8 (s, 3H) ; 1.25 (t, 3H) ; 2.1 (s, 3H) ; 2.3 (s, 3H) ; 3.7 (q, 2H) ; 5.85 (s, 1H) ; 5.9 (s, 1H)
G/ 17α-acetoxy-3-ethoxy-6-formyl-15β,16β-methylene-20-oxo-19-nor-pregna-3,5-diene (9.I.b : R₃ = H, R₄ = Ac, R₅ = H, alkyl = ethyl, n = 1)
   9.I.b was obtained by following the same process than 9.I.a from 8.I.b (yield : 93 %), mp : 199°C.
      IR (KBr, cm⁻¹) : 1721 νC = O ; 1645-1607 νC = C
      ¹H-NMR (CDCl₃,δ) : 0.65 (m, 1H) ; 0.75 (s, 3H) ; 1.35 (t, 3H) ; 2.1 (s, 3H) ; 2.3 (s, 3H) ; 3.9 (m, 2H) ; 6.35 (s, 1H) ; 10.25 (s, 1H)
H/ 17α-acetoxy-15β,16β-methylene-6-methylidene-3,20-dioxo-19-nor-pregna-4-ene (10.I.b : R₃ = H, R₄ = Ac, R₅ = H, n = 1)
   10.I.b was obtained by the same process than 10.I.a from 9.I.b (yield : 93 %), mp : 212°C.
      IR (KBr, cm⁻¹) : 1732 νC = O ; 1717 νC = O ; 1659 νC = O ; 1625-1585 νC = C
      ¹H-NMR (CDCl₃,δ) : 0.75 (m, 1H) ; 0.8 (s, 3H) ; 2.1 (s, 3H) ; 2.4 (s, 3H) ; 5-5.2 (2s, 2H) ; 6.15 (s, 1H)
I/ 17α-acetoxy-6-methyl-15β,16β-methylene-3,20-dioxo-19-nor-pregna-4,6-diene (11.I.b : R₃ = H, R₄ = Ac, R₅ = H, n = 1)
   11.I.b was obtained from 10.I.b as described for compound 11.I.a (yield : 75 %), mp : 205°C.
      IR (KBr, cm⁻¹) : 1724 νC = O ; 1720 νC = O ; 1662 νC = O ; 1624-1576 νC = C
      ¹H-NMR (CDCl₃,δ) : 0.75 (m, 1H) ; 0.85 (s, 3H) ; 1.9 (s, 3H) ; 2.1 (s, 3H) ; 2.35 (s, 3H) ; 6 (s, 1H) ; 6.3 (s, 1H).

### EXAMPLE 7

### 17α-acetoxy-6α-methyl-15β,16β-methylene-3,20-dioxo-19-nor-pregna-4-ene (12.I.b : R₁ = CH₃, R₃ = H, R₄ = Ac, R₅ = H, n = 1)

12.I.b was prepared following the same process than 12.I.a from 11.I.b (yield : 20 %), mp : 159°C.
   IR (KBr, cm⁻¹) : 1724 νC = O ; 1722 νC = O ; 1674 νC = O ; 1604 νC = C
   ¹H-NMR (CDCl₃,δ) : 0.7 (m, 1H) ; 1.18 (d, 3H) ; 2.1 (s, 3H) ; 2.35 (s, 3H) ; 5.9 (s, 1H)

### EXAMPLE 8

### 17α-ethoxy-15β,16β-methylene-6α-methyl-3,20-dioxo-19-nor-pregna-4-ene (12.I.c : R₁ = CH₃, R₃ = H, R₄ = C₂H₅, R₅ = H, n = 1)

To a stirred suspension of 12.I.b (1.2 g, 3.1 mmol.) in ethanol was added dropwise a solution of NaOH in water (10 %) until the pH was greater than 10 and the mixture was refluxed for 4 hours. Then, the mixture was extracted with CH₂Cl₂ and washed with water. The solvent was removed in vacuo and the crude product was purified by column chromatography on silica gel (toluene/AcOEt 6.5/3.5) to give 0.62 g of 17α-hydroxy-15β,16β-methylene-6α-methyl-19-nor-3,20-dione-pregna-4-ene (yield : 70 %).
IR (KBr, cm⁻¹) : 1706 νC = O ; 1668 νC = O ; 1610 νC = C ; 3446 νO-H
To a stirred mixture of 17α-hydroxy-15β,16β-methylene-6α-methyl-19-nor-3,20-dione- pregna-4-ene (6.2 g, 18 mmol.) and KOH (37 mmol.) in DMSO (60 mL) at 23°C was added dimethylsulfate (5 mL, 36 mmol.). After 2 hours, the solution was poured into cold water (500 mL). The crude product was filtered, dissolved in acetone (100 mL) and HCl (20 %) was added to the solution until the pH was 1. After 2 hours, the reaction mixture was concentrated in vacuo, extracted with CH₂Cl₂, washed with water and concentrated to give a residue which was flash-chromatographed on silica gel (toluene/AcOEt 9/1) to give 2 g of a crude product which was crystallized from isopropylether (yield : 10 %), mp : 147°C.
¹H-NMR (CDCl₃,δ) : 0.55 (m, 1H) ; 0.75 (s, 3H) ; 1.15 (d, 3H) ; 1.25 (t, 3H) ; 2.2 (s, 3H) ; 3.15 (m, 1H) ; 3.65 (m, 1H) ; 5.9 (s, 1H).

### EXAMPLE 9

### 17α-ethoxy-15β,16β-methylene-6-methyl-20-oxo-19-nor-pregna-4,6-diene (11.I.c: R₃ = H, R₄ = C₂H₅, R₅ = H, n = 1)

3,17α-diethoxy-15β,16β-methylene-6-methyl-19-nor-20-one-pregna-3-5(6)-diene was obtained from 12.I.c following the same process than 8.I.a (yield : 88 %).
¹H-NMR (CDCl₃,δ) : 0.5 (m, 1H) ; 0.7 (s, 3H) ; 1.2 (t, 3H) ; 1.7 (s, 3H) ; 2.15 (s, 3H) ; 3.15 (m, 1H) ; 3.6 (q, 2H) ; 3.8 (m, 1H) ; 5.45 (s, 1H).

To a stirred solution of the previous compound (2.6 g, 6.5 mmol.) in a mixture of acetone (60 mL) and water (1.6 mL) was added DDQ (1.7 g, 7.7 mmol.). After 20 min., the reaction mixture was concentrated in vacuo. The residu was flash-chromatographed twice (toluene/AcOEt : 8/2). Recrystallization from isopropylether gave 175 mg of 11.I.c (yield : 7 %), mp : 185°C.
¹H-NMR (CDCl₃,δ) : 0.6 (m, 1H) ; 0.8 (s, 3H) ; 1.2 (t, 3H) ; 1.9 (s, 3H) ; 2.2 (s, 3H) ; 3.2 (m, 1H) ; 3.7 (m, 1H) ; 5.9 (s, 1H) ; 6.3 (s, 1H).

### EXAMPLE 10

### 17α-acetoxy-6α,7α-dimethyl-15β,16β-methylene-3,20-dioxo-19-nor-pregna-4-ene (14.I.a : R₂ = CH₃, R₃ = H, R₄ = Ac, R₅ = H, n = 1)

To a stirred suspension of CuI (13.3 g, 70 mmol.) in Et₂O (135 mL) at 0°C was added a solution of MeLi (1.6 M) in Et₂O (88 mL, 140 mmol.). After 0.5 hour, a solution of 11.I.b (5.4 g, 14 mmol.) in THF (70 mL) was added dropwise to the mixture at -5°C over 0.5 hour. The reaction was quenched by addition of a NH₄Cl solution (1000 mL). The cuprous salts were filtered on Celite® and the filtrate was extracted by toluene, washed with water and concentrated in vacuo. The residue was flash-chromatographed (toluene/AcOEt : 9/1) and the crude product was recrystallized from Et₂O to give pure 17α-acetoxy-6,7α-dimethyl-15β,16β-methylene-19-nor-3,20-dioxo-pregna-5(6)-ene (yield : 7 %).
¹H-NMR (CDCl₃,δ) : 0.55 (m, 1H) ; 0.7 (s, 3H) ; 0.9 (d, 3H) ; 1.6 (s, 3H) ; 2.05 (s, 3H) ; 2.1 (s, 3H)

A solution of the previous compound (0.4 g, 1 mmol.) in a mixture of acetone (10 mL) and water (5 mL) was acidified with concentrated HCl until the pH was 1. After 6 hours, the reaction was concentrated, extracted with CH₂Cl₂, washed with water and the solvent was removed. The crude product was chromatographed on silica gel (toluene/AcOEt : 7/3 v/v) to give 0.2 g of the title compound (yield : 50 %), mp : 170°C.
¹H-NMR (CDCl₃,δ) : 0.7 (m, 1H) ; 0.75 (d, 3H) ; 0.85 (s, 3H) ; 1.1 (d, 3H) ; 2.1 (s, 3H) ; 2.3 (s, 3H) ; 5.85 (s, 1H).

### EXAMPLE 11

### 17α-acetoxy-6α,7α-dimethyl-3,20-dioxo-19-nor-pregna-4-ene (14.I.b: R₂ = CH₃, R₃ = H, R₄ = Ac, R₅ = H, n = 0)

17α-acetoxy-3-ethoxy-6-methyl-19-nor-20-one-pregna-3-5(6)-diene was obtained from 17α-acetoxy-6α-methyl-3,20-dioxo-19-nor-pregna-4-ene prepared using the procedures described in DE-A-2 148 261 following the same process than for 8.I.a (yield : 90 %).
IR (KBr, cm⁻¹) : 1730 νC = O ; 1652-1621 νC = C
¹H-NMR (CDCl₃,δ) : 0.6 (s, 3H) ; 1.3 (t, 3H) ; 2.0 (s, 3H) ; 2.1 (s, 3H) ; 3.75 (m, 2H) ; 5.4 (s, 1H).

This compound (15 g, 3.75 mmol.) was dissolved in acetone/water and treated with DDQ as described before for 11.I.c After a flash-chromatography (toluene/AcOEt 7/3) to remove the hydroquinone, the crude product (3.7 g) was used without other purification and following the process described for 14.I.a, 0.6 g of pure 17α-acetoxy-6,7α-dimethyl-19- nor-3,20-dioxo-pregna-5(6)-ene was obtained (yield : 4 %).
¹H-NMR (CDCl₃,δ) : 0.7 (s, 3H) ; 0.9 (d, 3H) ; 1.6 (s, 3H) ; 2.05 (s, 3H) ; 2.1 (s, 3H)

Compound 14.I.b was obtained from the previous compound following the same process than for 14.I.a (yield : 30 %), mp : 222°C.
¹H-NMR (CDCl₃,δ) : 0.65 (d, 3H) ; 0.7 (s, 3H) ; 1.2 (d, 3H) ; 2.0 (s, 3H) ; 2.1 (s, 3H) ; 5.8 (s, 1H).

### EXAMPLE 12

### 17α-acetoxy-7α-ethyl-6α-methyl-3,20-dioxo-19-nor-pregna-4-ene (14.I.c : R₂ = C₂H₅, R₃ = H, R₄ = Ac, R₅ = H, n = 0)

Using the procedure described for 14.I.b but replacing methyl lithium by ethyl magnesium chloride in the alkylation step, compound 14.I.c was obtained in 17 % yield, mp : 200° C.
¹H-NMR (CDCl₃, δ) : 0.70 (s, 3H) ; 0.98 (m, 3H) ; 1.12 (d, 3H) ; 2.05 (s, 3H) ; 2.15 (s, 3H) ; 2.60 (m, 1H) ; 5.89 (s, 1H).

### EXAMPLES 13-14

### 17α-acetoxy-3-(Z and E)-hydroxyimino-6α,7α-dimethyl-20-oxo-19-nor-pregna-4-ene (13.I.b : R₂ = CH₃, R₃ = H, R₄ = Ac, R₅ = H, n = O)

To a solution of 17α-acetoxy-6α,7α-dimethyl-3,20-dioxo-19-nor- pregna-4-ene (2 g, 5.18 mmol.) in dioxane (100 mL) was added successively hydroxylamine hydrochloride (0.755 g, 10.36 mmol.) and pyridine (5 mL). The mixture was heated to reflux for 1 hour. Then, the reaction was poured into iced water and acidified with a 1N HCl solution. Extraction with methylene chloride and evaporation of the solvent gave 1.92 g of a crude product which was flash-chromatographed using toluene/AcOEt as eluting solvent.

The first product eluted was the E isomer and was crystallized from ethanol (0.650 g, yield: 31.3%), mp > 250°C (example 13).
¹H-NMR (CDCl₃, δ) : 0.647-0.652 (d, 3H) ; 0.69 (s, 3H) ; 1.071-1.10 (d, 3H) ; 2.05 (s, 3H) ; 2.12 (s, 3H) ; 2.95 (m, 2H) ; 5.88 (s, 1H) ; 8.25 (bs, 1H)

It was followed by the Z isomer which was crystallized from a mixture of absolute ethanol and diisopropyl ether (0.170 g, yield: 16.4 %), mp : 208°C (example 14).
¹H-NMR (CDCl₃, δ) : 0.635-0.671 (d, 3H) ; 0.688 (s, 3H) ; 1.11-1.15 (d, 3H) ; 2.05 (s, 3H) ; 2.123 (s, 3H) ; 2.95 (m, 1H) ; 6.548 (s, 1H).

### EXAMPLES 15-31

The following examples illustrate the preparation of representative pharmaceutical formulations containing a compound of formula (I) :

### Pharmaceutical formulations for oral administration

### EXAMPLE 15

### Tablets with delayed release.

Unit formulation for various dosages :

| | |
|---|---|
| Compound of formula (I) | 0.50 to 10.00 mg |
| Aerosil® 200 | 0.37 to 0.50 mg |
| Precirol® ATO 5 | 1.85 to 2.25 mg |
| Methocel® E4 | 55.00 to 70.00 mg |
| Avicel® PH 101 | 10.00 to 20.00 mg |
| Lactose qs for 1 tablet of | 185.00 to 200.00 mg |

### EXAMPLE 16

### Tablets with fast release.

Unit formulation for various dosages :

| | |
|---|---|
| Compound of formula (I) | 0.50 to 10.00 mg |
| Aerosil® 200 | 0.37 to 0.50 mg |
| Precirol® ATO 5 | 1.85 to 2.50 mg |
| Avicel® PH 102 | 50.00 to 70.00 mg |
| Explotab® or polyplasdone® XL | 5.00 to 25.00 mg |
| Lactose qs for 1 tablet of | 185.00 to 200.00 mg |

### EXAMPLE 17

### Tablets.

Unit formulation for various dosages :

| | |
|---|---|
| Compound of formula (I) | 0.50 to 10.00 mg |
| Aerosil® 200 | 0.30 to 0.50 mg |
| Compritol® | 1.50 to 3.00 mg |
| Avicel® PH 101 | 55.00 to 70.00 mg |
| Lactose qs for 1 tablet of | 185.00 to 200.00 mg |

### Capsules.

Unit formulation for various dosages :

| | |
|---|---|
| Compound of formula (I) | 0.50 to 10.00 mg |
| Oleic acid qs for 1 capsule | 250.00 to 260.00 mg |

Coating : gelatine, preservatives, glycerol

### Pharmaceutical formulations for vaginal administration

### EXAMPLE 18

### Vaginal gynaecologic capsule.

Unit formulation for a capsule :

| | |
|---|---|
| Compound of formula (I) | 0.50 to 15.00 mg |
| Vaseline | 150.00 to 200.00 mg |
| Sorbitol sesquioleate | 150.00 to 200.00 mg |
| Synthetic perhydrosqualène qs for 1 capsule of 1.85 g | |

Coating : gelatine, glycerol, preservatives for a soft capsule weighing 2.55 g

### EXAMPLE 19

### Vaginal suppository.

Unit formulation for a suppository :

| | |
|---|---|
| Compound of formula (I) | 0.50 to 15.00 mg |
| Witepsol® H35 or H37 qs for a suppository of 3.00 g | |

### EXAMPLE 20

### Vaginal suppository with slow release.

Unit formulation for a suppository of 3.00 g :

| | |
|---|---|
| Compound of formula (I) | 0.50 to 30.00 mg |
| Witepsol® H19 or H35 | 1.00 to 1.30 g |
| Suppocire® BM or NAI50 | 1.00 to 1.50 g |
| Precirol® | 0.00 to 0.20 g |

### Pharmaceutical formulations for cutaneous or gynaecologic use

### EXAMPLE 21

### Bioadhesive gel for cutaneous or gynaecologic use.

Formula for 100 g :

| | |
|---|---|
| Compound of formula (I) | 0.10 to 1.00 g |
| Polyethylene glycol | 0.00 to 6.00 g |
| Transcutol® | 0.00 to 6.00 g |
| Carboxypolyvinyl polymer | 0.50 to 1.00 g |
| Preservatives | 0.30 mg |
| Triethanolamine qs pH 6.5 Purified water qs for 100 g | |

### EXAMPLE 22

### Gel for cutaneous use.

Formula for 100 g :

| | |
|---|---|
| Compound of formula (I) | 0.10 to 2.00 g |
| Polyethylene glycol or Transcutol® | 1.00 to 4.00 g |
| Ethyl alcohol | 20.00 to 40.00 g |
| Carboxypolyvinyl polymer | 0.50 to 2.00 g |
| Triethanolamine qs pH 6.5 Purified water qs for 100 g | |

### EXAMPLE 23

### Patches.

Content of the reservoir or matrix.
Preparation for 100 g

| | |
|---|---|
| Compound of formula (I) | 0.25 to 20.00 mg |
| Enhancer* | 0.20 to 0.50 g |
| Suspending agent (HPMC** or Aerosil®) | 0.10 to 1.00 g |
| Ethyl alcohol or silicone oil qs for 100 g | |

| | |
|---|---|
| * enhancer = isopropyl palmitate, propyleneglycol, menthol, azone, N,N-dimethylacetamide, mono- or disubstituted pyrrolidone derivatives ; | |
| **HPMC = hydroxypropylmethyl cellulose | |

### Pharmaceutical formulations for percutaneous administration

### EXAMPLE 24

### Implants.

Formulation for 100 g of material to be extruded :

| | |
|---|---|
| Compound of formula (I) | 1.00 to 5.00 g |
| Polymers (EVA, Polyorthocarbonates) qs for 100 g | |

The temperature of the mixture shall not exceede 150°C in order not to impair the active ingredient.

### Implants with reservoir.

The implant is a sealed silastic tubing having a length of 2.5 to 3.5 cm, a thickness of 0.4 to 0.8 mm and a diameter of 1.40 to 2mm. The preparation is formulated as a suspension as follows :
For 100 g of suspension.

| | |
|---|---|
| Compound of formula (I) | 30.00 to 50.00 g |
| Suspending agent qs for 100 g | |

50 mg of the suspension for one implant.

### EXAMPLE 25

### Injectible depot.

Unit formulation for a flask of 5 ml.

| | |
|---|---|
| Compound of formula (I) | 10.00 to 50.000 mg |
| Polyethylene glycol 4000 | 100.00 to 200.000 mg |
| Preservatives | 0.006 mg |
| Sodium chloride and citrate | 0.150 mg |
| Distilled water for injection qs for 5.00 ml | |

### EXAMPLE 26

### Injectible suspension.

Unit formulation for a 2 ml ampoule :

| | |
|---|---|
| Compound of formula (I) | 5.00 to 10.00 mg |

### Suspension solution

| | |
|---|---|
| Polysorbate® 80 | 0.015 g |
| Sodium carboxymethyl cellulose | 0.010 g |
| Sodium chloride | 0.010 g |
| Purified water for injection qs for 2.00 ml | |

### EXAMPLE 27

### Intra-uterine device with reservoir.

Device with a silastic reservoir having a length of 2.5 to 3.5 cm and a thickness of 0.4 to 0.8 mm. The preparation is formulated as a suspension as follows : For 100 g of suspension.

| | |
|---|---|
| Compound of formula (I) | 0.60 to 1.00 g |
| suspended to : | |
| Suspending agent (Aerosil® or HPMC) | 0.50 g |
| Synthetic perhydrogenalene qs for 100 g | |

### EXAMPLE 28

### Bioadhesive gynaecological foam.

Formula for a dispenser of 50 g and a spray valve (2 ml)

| | |
|---|---|
| Compound of formula (I) | 0.10 to 0.25 g |
| Carboxypolyvinyl polymer | 0.50 to 1.00 g |
| Isobutane | 5.00 to 10.00 g |
| Excipient base F25/1 qs for 50.00 g | |

Shake the suspension before use.
Dispensed dosage from 2.00 to 10.00 mg

### Pharmaceutical formulation for nasal administration

### EXAMPLE 29

### Nasal suspension.

Formulation for 100 g of suspension :

| | |
|---|---|
| Compound of formula (I) | 5.00 to 50.00 mg |
| Aerosil® PH 101 | 10.00 to 20.00 mg |
| Sodium carboxymethyl cellulose | 5.00 to 50.00 mg |
| Phenylethyl alcohol | 2.00 to 10.00 mg |
| Polysorbate® 80 | 10.00 to 20.00 mg |
| Purified water qs for 100 g | |

Shake the suspension before use
Dispensed dosage from 0.5 to 2.5 mg

### Pharmaceutical formulations for ophtalmic administration

### EXAMPLE 30

### Ophtalmic solution (collyrium).

Formulation for 100 g of solution. Container of 5 ml with glass droppers :

| | |
|---|---|
| Compound of formula (I) | 0.50 to 1.00 g |
| Glycerol | 5.00 g |
| Polyvidone or sodium chloride | 0.50 to 0.90 g |
| Sorbitol | 4.00 g |
| Preservatives (benzalkonium chloride or Cetrimide®) | 0.01 g |
| EDTA | 0.01 g |
| Distilled water qs for 100 g | |

The solution is a sterile aqueous solution ; it may contain stabilisers and antimicrobial agents.

The recommended dose is one drops four times daily.

### EXAMPLE 31

### Ophtalmic gel.

Formulation for 100 g of gel. Container : collapsible tube

| | |
|---|---|
| Compound of formula (I) | 0.50 to 2.00 g |
| Cetrimide® | 0.01 g |
| Sorbitol | 4.00 g |
| EDTA | 0.01 g |
| Carboxypolyvinyl polymers (Carbopol® 971) | 0.14 to 0.20 g |
| Sodium hydroxyde 10 % qs pH 6.5 Purified water qs for 100 g. | |

The sterile aqueous gel is packed in a collapsible tube.

The recommended dose is one drop one or two times daily.

### Typical examples of the compounds of formula (I) provided by this invention include :

. 17α-acetoxy-6,15β-dimethyl-3,20-dioxo-19-nor-pregna-4,6-diene
. 17α-acetoxy-6α,15β-dimethyl-3,20-dioxo-19-nor-pregna-4-ene
. 17α-acetoxy-6β,15β-dimethyl-3,20-dioxo-19-nor-pregna-4-ene
. 17α-acetoxy-6α,15β-dimethyl-3-E-hydroxyimino-20-oxo-19-nor-pregna-4-ene
. 17α-acetoxy-6α,15β-dimethyl-3-Z-hydroxyimino-20-oxo-19-nor-pregna-4-ene
. 17α-acetoxy-6-methyl-15β,16β-methylene-3,20-dioxo-19-nor-pregna-4,6-diene
. 17α-acetoxy-6α-methyl-15β,16β-methylene-3,20-dioxo-19-nor-pregna-4-ene
. 17α-ethoxy-15β,16β-methylene-6α-methyl-3,20-dioxo-19-nor-pregna-4-ene
. 17α-ethoxy-15β,16β-methylene-6-methyl-20-oxo-19-nor-pregna-4,6-diene
. 17α-acetoxy-6α,7α-dimethyl-15β,16β-methylene-3,20-dioxo-19-nor-pregna-4-ene
. 17α-acetoxy-7α-ethyl-6α-methyl-3,20-dioxo-19-nor-pregna-4-ene
. 17α-acetoxy-6α,7α-dimethyl-3,20-dioxo-19-nor-pregna-4-ene
. 17α-acetoxy-6α-methyl-7α-ethyl-3,20-dioxo-19-nor-pregna-4-ene
. 17α-acetoxy-3-Z-hydroxyimino-6α,7α-dimethyl-20-oxo-19-nor-pregna-4-ene
. 17α-acetoxy-3-E-hydroxyimino-6α,7α-dimethyl-20-oxo-19-nor-pregna-4-ene

## Claims

1. A compound having the following general formula (I): 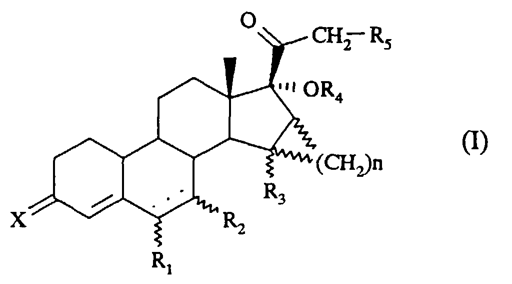 wherein :
each of R₁ and R₂ is hydrogen or a (C₁-C₆)alkyl, R₁ and R₂ being not simultaneously hydrogen ;
R₃ is hydrogen, a (C₁-C₆)alkyl or a (C₁-C₆)alkoxy ;
R₄ is hydrogen, a (C₁-C₆)alkyl or a group -COR₆ where R₆ is a (C₁-C₆)alkyl ;
R₅ is hydrogen or a (C₁-C₆)alkyl ;
n is zero or one ;
X is oxygen or an hydroxyimino group ; and
the dotted line may represent a double bond ; provided that when n = 0, R₃ is hydrogen only if both R₁ and R₂ are a (C₁-C₆)alkyl.

2. A compound according to claim 1, wherein R₁ and R₃ are a (C₁-C₆)alkyl, R₂ and R₅ are hydrogen, R₄ is a group -COR₆, n is zero and R₆ and X are as defined for (I) in claim 1.

3. A compound according to claim 1, wherein R₁ is a (C₁-C₆)alkyl, R₃ and R₅ are hydrogen, R₄ is a group -COR₆, n is one, and X, R₂ and R₆ are as defined for (I) in claim 1;

4. A compound according to claim 1, wherein R₁ and R₄ are a (C₁-C₆)alkyl, R₂, R₃ and R₅ are hydrogen, n is one and X is as defined for (I) in claim 1.

5. A compound according to claim 1, wherein R₁ and R₂ are a (C₁-C₆)alkyl, R₃ and R₅ are hydrogen, R₄ is a group -COR₆, n is zero and R₆ and X are as defined for (I) in claim 1.

6. A pharmaceutical composition containing (i) an effective amount of a compound of formula (I) according to any one of claims 1-5 and (ii) suitable excipients.

7. A pharmaceutical composition according to claim 6, which contains from 0.01 wt% to 99.99 wt% of the compound of formula (I).

8. A pharmaceutical composition according to claim 6 or 7, which is a contraceptive composition.

9. A contraceptive composition according to claim 8, which further contains an effective amount of an oestrogen.

10. Use of a compound of formula (I) according to any one of claims 1-5 for the preparation of a medicament intended for treating or preventing gynaecological disorders associated to an oestrogen/progesterone imbalance.

11. Use of a compound of formula (I) according to any one of claims 1-5 for the preparation of a medicament intended for inhibiting oestrogen transformation of endometrium.

12. Use of a compound of formula (I) according to any one of claims 1-5 for the preparation of a medicament intended for inhibiting gonadotropic/gonadal secretions.

13. Use of a compound of formula (I) according to any one of claims 1-5, alone or in combination with an oestrogen, for the preparation of a contraceptive agent.

14. Use of a compound of formula (I) according to any one of claims 1-5, alone or in combination with an oestrogen for the preparation of a medicament intended for postmenopausal hormone replacement therapy.
